# EUROPEAN PATENT APPLICATION

(11) **EP 2 088 204 A1**
(43) Date of publication of application: **12.08.2009**
(21) Application number: 08002127.2
(22) Date of filing: 05.02.2008
(51) Int. Cl.: C12Q 1/68

(54) **Method and kit for diagnosing or controlling the treatment of ovarian cancer**

(71) Applicant: Adnagen AG, 30853 Hannover-Langenhagen (DE)
(72) Inventor: Albert, Winfried, Dr., 82377 Penzberg (DE); Hauch, Siegfried,Dr., 31863 Coppenbrügge (DE); Lustig, Michael, Dr., 27221 Ritterhude (DE)
(74) Representative: Pfenning, Meinig & Partner GbR

(57) **Abstract**

The present invention pertains to a procedure and a kit for the diagnosis or monitoring of ovarian cancer in humans. This procedure is based on the feature that one recognizes the presence or absence in a human blood sample of at least two different mRNAs that code for various members of the group comprising tumor marker proteins EGFR, CEA, CK20, GA733-2, MUC1, HER2, MUC4, and CA12-5 and a conclusion is drawn from this in regard to the presence of ovarian carcinoma cells in the blood sample, and thus in regard to possible metastasis.

SEQUENCE LISTING

<110>  AdnaGen AG

<120>  Method and kit for diagnosing or controlling the treatment of
       ovarian cancer

<130>  079EP1943

<160>  16

<170>  PatentIn version 3.3

<210>  1
<211>  24
<212>  DNA
<213>  Artificial

<220>
<223>  Primer

<400>  1
aatcgrcaat gccagtgtac ttca                                            24


<210>  2
<211>  24
<212>  DNA
<213>  Artificial

<220>
<223>  Primer

<400>  2
taacgcgttg tgatctcctt ctga                                            24


<210>  3
<211>  24
<212>  DNA
<213>  Artificial

<220>
<223>  Primer

<400>  3
agtcgggctc tggaggaaaa gaaa                                            24


<210>  4
<211>  24
<212>  DNA
<213>  Artificial

<220>
<223>  Primer

<400>  4
gatcataatt cctctgcaca tagg                                            24


<210>  5
<211>  24
<212>  DNA
<213>  Artificial

<220>
<223>  Primer

<400>  5
agaaatgacg caagagccta tgta                                              24

<210>  6
<211>  24
<212>  DNA
<213>  Artificial

<220>
<223>  Primer

<400>  6
aacttgtgtg tgttgctgcg gtat                                              24

<210>  7
<211>  24
<212>  DNA
<213>  Artificial

<220>
<223>  Primer

<400>  7
tcagcttcta ctctggtgca caac                                              24

<210>  8
<211>  24
<212>  DNA
<213>  Artificial

<220>
<223>  Primer

<400>  8
tggtagtagt cggtgctggg atct                                              24

<210>  9
<211>  24
<212>  DNA
<213>  Artificial

<220>
<223>  Primer

<400>  9
cccagtgtgt caactgcagc cagt                                              24

<210>  10
<211>  24
<212>  DNA
<213>  Artificial

<220>
<223>  Primer

<400>  10
cagatgggca tgtaggagag gtca                                              24

<210>  11
<211>  20
<212>  DNA

<213>  Artificial

<220>
<223>  Primer

<400>  11
tcaacgcctc ggtggcatac                                                    20


<210>  12
<211>  20
<212>  DNA
<213>  Artificial

<220>
<223>  Primer

<400>  12
ctgtcacatc gcgcacgtct                                                    20


<210>  13
<211>  24
<212>  DNA
<213>  Artificial

<220>
<223>  Primer

<400>  13
acacattccg cttctgcctg gtca                                               24


<210>  14
<211>  19
<212>  DNA
<213>  Artificial

<220>
<223>  Primer

<400>  14
ccccggtgtg gtgcctgtt                                                     19


<210>  15
<211>  24
<212>  DNA
<213>  Artificial

<220>
<223>  Primer

<400>  15
ctggagaaga gctacgagct gcct                                               24


<210>  16
<211>  24
<212>  DNA
<213>  Artificial

<220>
<223>  Primer

<400>  16
acaggactcc atgcccagga agga                                               24

## Description

The present invention pertains to a procedure and a kit for the diagnosis or monitoring of ovarian cancer in humans.

The ability to detect a relapsing malignant tumor in a timely manner via the occurrence of metastasizing tumor cells in the blood is relevant in cancer after-care. So-called "tumor markers" are determined quantitatively at the protein level (immunologically or enzymatically) in the blood, or in other bodily fluids, in cancer patients when using current test methods.

These detection procedures are suitable only to a limited extent for tumor diagnosis, or monitoring/after-care, since increased tumor marker values can also be produced by non-tumor diseases (e.g. inflammation of the gastrointestinal tract, cirrhosis of the liver, viral infections), heavy smoking, or as a result of pregnancy.

The currently used diagnostic methods are inexact when one is dealing with the evaluation of the malignant potency of residual tumors after chemotherapy has been carried out in the metastasizing stages. Some clinical studies indicate the prognostic importance of disseminated tumor cells. However, numerous methodological aspects are critical and have not been adequately standardized so far. Thus, detection methods for occult-or residual-metastasis have to be found that permit timely classification into the various primarily curative therapeutic options.

The effort to improve the chances of healing is currently accompanied, on the one hand, by the search for and the use of new tumor markers and, on the other hand, by increasing sensitivity of the methods that are used.

The problem of the present invention is to make available a procedure and a kit with which the diagnosis or monitoring of ovarian cancer is possible in a simple, safe and repeatable manner.

This problem is solved by the procedure in accordance with Claim 1 and with the kit in accordance with Claim 30 and the microarray in accordance with Claim 48. Advantageous further developments of the procedure, of the kit, and of the microarray are given in the pertinent dependent claims.

In accordance with the invention, the presence or absence of at least two different mRNAs from the group comprising tumor marker proteins EGFR, CEA, GA733-2, MUC1, HER2, CA12-5 and MUC4 are recognized in a human blood sample by means of the kit in accordance with the invention or by means of the procedure in accordance with the invention. This means selection of any particular combination of two tumor markers named above and recognition of their mRNA in a blood sample. Although at least two of the markers mentioned above are detected according to the present invention, any further marker, which is not mentioned here, may or may not be detected additionally.

Since the mRNAs of the markers that have been described are not normally present in expressed form in the blood of healthy persons, a direct correlation is found between a positive RT-PCR result detection for these tumor markers and circulating tumor cells in the blood that can lead to metastasis.

Since individual markers can be expressed differently in a therapy-dependent manner, and ovarian cancer exhibits pronounced heterogeneity in the expression pattern of the ovarian cancer cells, it is expedient to examine a combination of tumor markers in order to recognize all the tumor cells that are circulating in the blood. As a result of this, tumor cells can also be recognized when the expression of a particular marker is relatively slight in a patient or in a stage of the disease, which could otherwise lead to a supposedly negative result. However, the use of additional markers usually encounters limits if mononuclear blood cells exhibit background expression ("illegitimate transcription") that impedes exact analysis.

Thus the following advantageous combination of markers is proposed in accordance with the invention for the recognition of ovarian cancer cells.
At least the two markers in at least one of the following groups:
GA733-2 and MUC1;
GA733-2 and HER2;
GA733-2 and CEA;
GA733-2 and CA12-5;
GA733-2 and EGFR;
GA733-2 and MUC4;
   or
at least the three markers in at least one of the
following groups:
GA733-2, MUC1 and HER2;
GA733-2, MUC1 and CA12-5;
GA733-2, MUC1 and EGFR;
GA733-2, MUC1 and CEA;
GA733-2, MUC1 and MUC4;
GA733-2, CA12-5 and EGFR;
GA733-2, CA12-5 and CEA;
GA733-2, CA12-5 and HER2;
GA733-2, CA12-5 and MUC4;
GA733-2, HER2 and EGFR;
GA733-2, HER2 and CEA;
GA733-2, HER2 and MUC4;
GA733-2, EGFR and CEA;
GA733-2, EGFR and MUC4;
GA733-2, CEA and MUC4;
   or
at least the markers in at least one of the following groups of marker combinations
GA733-2, HER2, CEA and EGFR;
GA733-2, HER2, MUC1 and CA12-5;
GA733-2, HER2, MUC1, CA12-5, EGFR and CEA;
GA733-2, MUC1, CA12-5 and EGFR;
GA733-2, HER2, MUC4 and EGFR;
GA733-2, HER2, MUC4 and CEA;
   or further combinations of at least markers comprising at least marker GA733-2 as first marker, at least one marker of the group of markers EGRF, CEA, MUC1, HER2, CA12-5 and MUC4 as second marker, at least one marker of the group of markers EGRF, CEA, MUC1, HER2, CA12-5 and MUC4 as third marker different from the second marker and at least one marker of the group of markers EGRF, CEA, MUC1, HER2, CA12-5 and MUC4 as forth marker different from the second marker and different from the third marker.

Advantageously, to each of the combinations of two, three or more markers cited in the preceeding text the further marker CA12-5 may be added.

Surprisingly, the combination of the three markers GA733-2, HER2 and MUC1, which was already successfully used in the *AdnaTest BreastCancer* kits for detection of circulating breast cancer cells, turned out to also be an advantageous combination of markers for the detection of circulating tumor cells in ovarian cancer. In contrast, the combination used in the *AdnaTest Colon Cancer* kits was much less effective. The combination of these three markers can be preferably supplemented by additional markers from the list of markers provided in claim 1, especially CA12-5.

Further, the combination of markers GA733-2, MUC1 and EGFR turned out to be a further most advantageous marker combination for detection of circulating tumor cells associated with ovarian cancer. This combination of markers may be supplemented by further markers as mentioned above.

Use can be made of the primers indicated in the following table for the amplification of segments of the markers.

| Tumor marker - primer | Sequence 5' → 3' | PCR product |
|---|---|---|
| GA733-2 sense | AATCGTCAATGCCAGTGTACTTCA | 395 bp |
| GA733-2 anti-sense | TAACGCGTTGTGATCTCCTTCTGA | |
| EGFR sense | AGTCGGGCTCTGGAGGAAAAGAAA | 163 bp |
| EGFR antisense | GATCATAATTCCTCTGCACATAGG | |
| CEA sense | AGAAATGACGCAAGAGCCTATGTA | 231 bp |
| CEA antisense | AACTTGTGTGTGTTGCTGCGGTAT | |
| MUC1 sense | TCAGCTTCTACTCTGGTGCACAAC | 299 bp |
| MUC1 antisense | TGGTAGTAGTCGGTGCTGGGATCT | |
| HER2 sense | CCCAGTGTGTCAACTGCAGCCAGT | 265 bp |
| HER2 antisense | CAGATGGGCATGTAGGAGAGGTCA | |
| MUC4 sense | TCAACGCCTCGGTGGCATAC | 296 bp |
| MUC4 antisense | CTGTCACATCGCGCACGTCT | |
| CA12-5 sense | ACACATTCCGCTTCTGCCTGGTCA | 432 bp |
| CA12-5 antisense | CCCCGGTGTGGTGCCTGTT | |
| | | |
| Internal control | | |
| Aktin sense | CTGGAGAAGAGCTACGAGCTGCCT | 111 bp |
| Aktin antisense | ACAGGACTCCATGCCCAGGAAGGA | |

The designations of the markers are explained in the following table.

| **Gene or gene product** | **Genre** |
|---|---|
| Human carninoma-associated antigen GA733-2 gene | GA733-2 |
| Human epidermal growth factor receptor (EGFR) gene | EGFR |
| Human carcinoembryonic antigen (CEA) gene | CEA |
| Homo sapiens mucin 1 (MUC1) | MUC1 |
| Homo sapiens C-erb B2/neu protein (ERBB2) gene | HER-2 |
| Homo sapiens mucin 16 (MUC16) | CA12-5 |
| Homo sapiens mucin 4 (MUC4) | MUC4 |

In the case of using RT-PCR systems for the detection of tumor cells, specificity is a critical point because of the very high amplification rate. The least contamination, e.g. via extraneous RNA or illegitimate transcription, can in this way falsify the result.

An increase in specificity as a result of concentrating the tumor cells relative to the blood cells and, at the same time, an increase in sensitivity in the detection of tumor cells can be achieved (detection rate of 1 tumor cell per 10⁷ mononuclear blood cells) by using immunocytochemistry with monoclonal antibodies that react against tumor cell antigens. In this way the tumor cells are separated by means of specific antibodies or an antibody mixture of mononuclear blood cells. Separation can take place by means of magnetic particles (Dynabeads, Dynal) to which the antibodies are bound. This is described in greater detail in the following section.

Eukaryotic cells carry a plurality of different molecules on their cell surface. The combination of expressed surface molecules differs depending on the origin and the function of the individual cell, so that patterns are produced that are cell-type specific. Antibodies are utilized in order to recognize these cell-type specific patterns. Antibodies bind with high specificity to their antigen, namely to selected surface molecules in this case. This property is utilized in order to recognize cells and to differentiate them from one another by means of specific antibody binding on the basis of their cell-type specific patterns.

The expression of special surface proteins differentiates tumor cells from non-transformed cells of this cell type. Since, in the case of tumor cells, this special pattern of surface antigens also differs from the patterns that are typical of blood cells, tumor cells can be differentiated in the blood. In order to identify tumor cells, antibodies that specifically recognize these special surface proteins are utilized as tools. This specific antibody binding becomes usable for various analyses and separation methods.

In addition to recognizing cells via their surface epitopes, it is also possible to separate recognized cells from non-recognized ones because of the intensive binding of immunoglobulins that are specially selected for this purpose.
1.Separation principle based on the liquid phase; e.g. continuous flow cytometry or fluorescence activated cell sorting
Antibodies are coupled to fluorescent dyes for the purpose of continuous flow cytometry analysis. Isolated cells are individually led past a light source (laser) in a constant stream of liquid. The fluorescent dyes bound to the antibodies are excited during illumination of the cells, and they irradiate light of a particular wavelength. The irradiated light is detected, and the measured signal is stored in digital form. The light signal can be assigned to individual cells. The antibody-labeled cell is recognized in this way, and can now be separated from other cells. The cells are isolated in extremely small drops for separation purposes. After recognizing the antibody-labeled cell, the drops in question are deflected into a collection container.
2. Separation principle based on the solid phase; e.g. magnetic separation:
Antibodies are coupled to magnetic or pseudomagnetic particles for the purpose of magnetic separation. After introducing the magnetic or pseudomagnetic particles to a magnetic field, the particles migrate in this magnetic field. During movement in this magnetic field, the cells to which the coupled antibodies are bound are carried along, and separated from other cells.
Thus, in order to recognize tumor cells by means of magnetic separation, antibodies are covalently coupled to magnetic or pseudomagnetic particles that possess a defined number of chemically activated sites on their surface. The separation specificity is determined by the specificity of the antibodies. A blood sample that contains tumor cells is mixed with antibody-coupled magnetic or pseudomagnetic particles; the particles and blood then move relative to one another. Those (tumor) cells which are recognized by the antibodies (that are bound to the solid phase) and which are firmly bound to them follow the movement of the particles. As a result, it is possible to withdraw, from the blood, the particles with the cells that are bound to them (e.g. toward the wall of the separation vessel) upon applying a magnetic or pseudomagnetic field. The blood that has been tumor-cell depleted in this way can be exchanged for other solutions, whereby the cells that have been separated via magnetic or pseudomagnetic particles remain behind, and are available for additional applications until the point in time of switching off/removing the magnetic field.
Use can advantageously be made of specific antibody mixtures in order to recognize the tumor cells, whereby these mixtures have either been optimized with respect to tumor cells in a general manner, or they have been specifically optimized with respect to breast cancer cells as well. For example, a combination of antibody against the epithelial cell marker Ber-EP4 with differnt other antibodies directed against another epitope of the same antigen (MOC31) or against different tumor cell associated antigens (anti-MUC1 antibodies, anti-EGFR antibodies, anti-CA12-5 antibodies) is used in order to increase the efficacy of the recognition and selection step and not to loose any but to recover most or all tumor cells present in the sample.
If a mixture of antibodies is used to recognize and select tumor cells, any cell marked by at least one of the antibodies can be selected in order to increase sensitivity of the recognition and selection step and select all cells which might be tumor cells.
Recognition that is specially directed toward ovarian cancer cells can be achieved via an additionally optimized antibody mixture in accordance with the table below. This is based on the selective expression of certain surface proteins, whereby ovarian cancer cells are differentiated from other cancer cells. Surprisingly, it was found, that the antibody combination used in the *AdnaTest BreastCancerSelect* kit turned out to be the most efficient one for selection of circulating ovarian cancer cells, whereas the antibody mix used in the *AdnaTest ColonCancerSelect* kit was inefficient, although both mixtures contained the same anti-EPCAM antibody.

| Antigen | Clone (Provider) | Antibody Concentration in 5 ml blood |
|---|---|---|
| EpCAM | 2D3 (AdnaGen AG) | 0.375 µg/100 µl beads |
| EpCAM | Moc31 (Acris) | 1.3125 µg/100 µl beads |
| HER2 | 10E9 (AdnaGen AG) | 0.625 µg/100 µl beads |
| MUC1 | HMPV (BD) | 2.0 µg/100 µl beads |

In comparison to the antibodies when used separately in each case, such antibody mixtures show increased sensitivity in terms of cell recognition and cell separation, independent of the method used.

The diagnosis kit in accordance with the invention and the procedure in accordance with the invention also make it possible to subsequently use the sorted and separated cells further as desired. For example, these can be inserted into a suitable cell culture medium where they can be cultivated in situ.

Since the cells are intact following separation, the properties of the cell membrane and of the cell nucleus are also conserved. This opens up the possibility of microscopically investigating the expression of additional surface markers, and of carrying out chromosome analyses as well. The sorted cells are applied to microscope slides for this purpose. The detection of additional surface markers can take place cytochemically or via fluorescence microscopy. Likewise, genetic analyses can be carried out such as, for example, chromosome analyses by means of FISH (fluorescence in situ hybridization), or via kary-ogram compilation or by gene expression analysis.

In the following, examples for detection and characterization of circulating tumor cells in patients with primary ovarian cancer are shown by the inventive method of molecular profiling.

### Patients and Methods

2 x 5 ml blood were obtained from ovarian cancer patients before (n=30) and after (n=23) completion of chemotherapy, CTC were immunomagnetically enriched from blood by targeting different epitopes of EpCAM and MUC1, which was followed by isolation of their mRNA with oligo-dT magnetic beads.

Enrichment was done using two different sets of antibodies, i.e.
set (*AdnaTest BreastCancerSelect*, Adnagen AG, Langenhagen, Germany) designated "BR" containing one antibody directed against GA733-2 (EpCAM) and two antibodies directed against MUC1 (HMPV and GP1.4).
set (*AdnaTest ColonCancerSelect*, Adnagen AG, Langenhagen, Germany) designated "CO" containing 2 antibodies directed against GA733-2 (EpCAM and MOC31).

In each assay, cells labeled by at least one of the antibodies were selected for enrichment.

The mRNA fraction was analyzed for EpCAM, MUC1, MUC4, CA12-5, HER-2, EGFR and CEA transcripts by multiplex RT-PCR with the *AdnaTests ColonCancerDetect* and *BreastCancerDetect* (AdnaGen AG), containing primers for amplification of transcripts of GA733-2, MUC1 and HER2 (*AdnaTest BreastCancerDetect* designated "BR") or GA733-2, CEA, EGFR (*AdnaTest ColonCancerDe*tect designated "CO").

CA12-5 and MUC4 transcripts are both assayed in a separate RT-PCR. The PCR products were analyzed by capillary electrophoresis on the Agilent Bioanalyzer 2100. Blood of healthy donors as controls was used to confirm the specificity of the tests.

In total, 52 patients with ovarian primary tumor were analyzed for circulating tumor cells (CTC) in samples of peripheral blood. In these patients, a total of 14 patients (27 %) were found positive for CTC.

The following table shows the detection results using specific combinations of the *AdnaTest CancerSelect* kits and the *AdnaTest CancerDetect* kits. In the table, the first designation (BR, CO) determines the immunomagnetic selection step and the second designation (BR, CO) determines the expression profiling step, e.g. BR-CO designation as a selection step using *AdnaTest BreastCancerSelect* and a profiling step using *AdnaTest ColonCancerDetect*.

| **Patient#** | **Max BR_BR** | **Max BR_CO** | **Max CO_CO** | **Max CO_BR** | **Max ALL** |
|---|---|---|---|---|---|
| 1 | 4.91 | 0.13 | 0 | 3.03 | 4.91 |
| 2 | 1.67 | 0 | 0 | 0.13 | 1.67 |
| 3 | 0 | 0 | 1.51 | 0 | 1.51 |
| 4 | 0.42 | 0 | 0 | 0.76 | 0.76 |
| 5 | 0.54 | - | - | - | 0.54 |
| 6 | 0.45 | 0.53 | 0.39 | 0 | 0.53 |
| 7 | 0.5 | - | - | - | 0.5 |
| 8 | 0.43 | 0 | 0 | 0.14 | 0.43 |
| 9 | 0.09 | - | - | 0.39 | 0.39 |
| 10 | 0.29 | - | - | - | 0.29 |
| 11 | 0 | 0.21 | 0.27 | 0 | 0.27 |
| 12 | 0 | 0 | 0.26 | 0 | 0.26 |
| 13 | 0.17 | - | - | - | 0.17 |
| 14 | 0.16 | 0 | 0 | 0.16 | 0.16 |
| | | | | | |
| Detected positive | 10 | 2 | 4 | 4 | 14 |
| Total positive | 14 | 9 | 9 | 10 | 14 |
| Percentage | 71 % | 22 % | 44 % | 40 % | 100% |

100% recovery is considered when all transcripts were tested for (Max ALL).

Surprisingly, already the use of *AdnaTest BreastCancerDetect* provides for a detection rate of at least 40 % and in combination with *AdnaTest BreastCancerSelect* a detection rate of 71 0.

Using *AdnaTest ColonCancerDetect*, no such detection rates could be obtained.

Obviously, it is the marker combination GA733-2, MUC1 and HER2 of *AdnaTest BreastCancerDetect* kit, which provides improved performance to detect CTC's in patients with primary ovarian cancer. This could further be improved by combination with an immunoseparation using antibodies for EpCAM, HMPV and GP1.4.

Sensitivity could be further improved by detection of additional patients if additionally to analysis with the *AdnaTest BreastCancerDetect* kit (containing markers GA733-2, MUC1 and HER2) the samples were analyzed for CA12-5 and/or EGFR transcripts as shown in the following table.

| | number of positiv patient samples out of 50 samples | percentage of positiv patient samples |
|---|---|---|
| *AdnaTest BreastCancerDetect* kit | 10 | 20 % |
| CA12-5 | 5 | 10 % |
| EGFR | 3 | 6 % |
| *AdnaTest BreastCancerDetect* kit + CA12-5 | 13 | 26 % |
| *AdnaTest BreastCancerDetect* kit + EGFR | 13 | 26 % |
| *AdnaTest BreastCancerDetect* kit + CA12-5 + EGFR | 16 | 32 % |

Therein samples from 50 ovarian cancer patients were tested for CTC using the *AdnaTest BreastCancer*Detect kit as described in the manufacturers instuctions. Additionally, the cDNA obtained from these samples was tested for expression of marker CA12-5 and/or expression of marker EGFR. Using *AdnaTest BreastCancerDetect* kit alone, 10 out of 50 patient samples (20%) were positive tested for CTC. Analysing expression of markers CA 12-5 or EGFR alone, 5 out of 50 patient samples (10%, CA12-5) and 3 out of 50 patient samples (6%, EGFR) were tested positive for CTC. However, evaluating the combination of *AdnaTest BreastCancerDetect* kit and CA12-5, i.e. considering the sample to be positive if only one of the markers contained in this marker combination turned out to be positive, 13 out of 50 patient samples (26 %) were tested positive for CTC. Evaluating the combination of *AdnaTest BreastCancerDetect* kit and EGFR, also 13 out of 50 patient samples (26 %) were tested positive for CTC. Evaluating the combination of *AdnaTest BreastCancerDetect* kit, EGFR and CA12-5, 16 out of 50 patient samples (32 %) were tested positive for CTC. This is a further major improvement in detection rate of CTC in peripheral blood of patients with ovarian cancer.

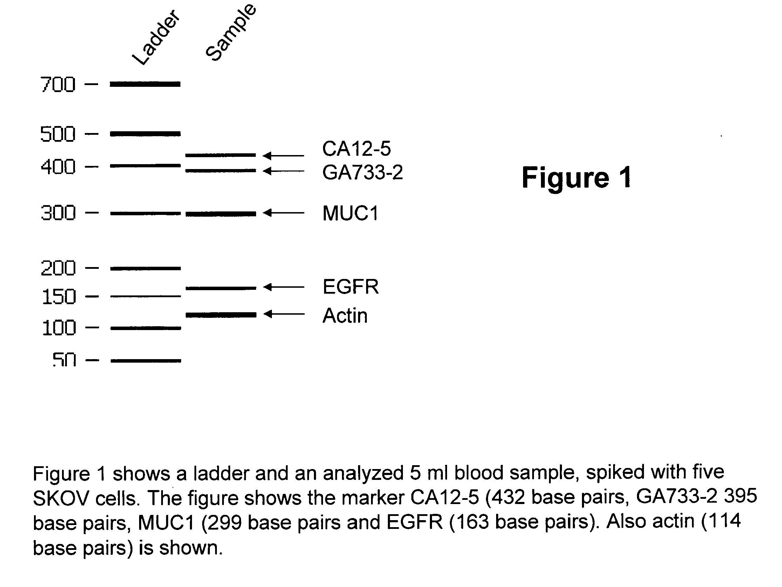

Further, superior sensitivity was found by detecting a combination of markers GA733-2, MUC1, EGFR and CA12-5. This is demonstrated in fig. 1. Fig. 1 shows an electrophoresis gel with a ladder and an analyzed sample of peripheral blood. Therein 5 SKOV cells were spiked into a sample volume of 5 ml. Fig. 1 further shows bands for markers CA12-5 at 432 base pairs, GA733-2 at 395 base pairs, MUC1 at 299 base pairs, EGFR at 163 base pairs and actin at 114 base pairs. Any of these markers tested positive in this specific sample, allowing for even higher sensitivity than 5 cells per 5 ml peripheral blood.

## Claims

1. Procedure for the diagnosis or monitoring of ovarian cancer in humans, **characterized in that** the presence or absence of at least two different mRNAs is recognized in a human blood sample, whereby these mRNAs code for at least two members of the group comprising tumor marker proteins EGFR, CEA, GA733-2, MUC1, HER2, CA12-5 and MUC4 and conclusions are drawn from this in regard to the presence of ovarian carcinoma cells in the sample of blood, and hence in regard to possible metastasis.

2. Procedure in accordance with Claim 1, **characterized in that** the presence or absence is recognized of members of at least one group of the following groups of marker:
GA733-2 and MUC1;
GA733-2 and HER2;
GA733-2 and CEA;
GA733-2 and CA12-5;
GA733-2 and EGFR;
GA733-2 and MUC4;
or
GA733-2, MUC1 and HER2;
GA733-2, MUC1 and CA12-5;
GA733-2, MUC1 and EGFR;
GA733-2, MUC1 and CEA;
GA733-2, MUC1 and MUC4;
GA733-2, CA12-5 and EGFR;
GA733-2, CA12-5 and CEA;
GA733-2, CA12-5 and HER2;
GA733-2, CA12-5 and MUC4;
GA733-2, HER2 and EGFR;
GA733-2, HER2 and CEA;
GA733-2, HER2 and MUC4;
GA733-2, EGFR and CEA;
GA733-2, EGFR and MUC4;
GA733-2, CEA and MUC4;
or
GA733-2, HER2, CEA and EGFR;
GA733-2, HER2, MUC1 and CA12-5;
GA733-2, HER2, MUC1, CA12-5, EGFR and CEA;
GA733-2, MUC1, CA12-5 and EGFR;
GA733-2, HER2, MUC4 and EGFR;
GA733-2, HER2, MUC4 and CEA;
or further combinations of at least markers comprising at least marker GA733-2 as first marker, at least one marker of the group of markers EGRF, CEA, MUC1, HER2, CA12-5 and MUC4 as second marker, at least one marker of the group of markers EGRF, CEA, MUC1, HER2, CA12-5 and MUC4 as third marker different from the second marker and at least one marker of the group of markers EGRF, CEA, MUC1, HER2, CA12-5 and MUC4 as forth marker different from the second marker and different from the third marker.

3. Procedure in accordance with one of the preceding claims, **characterized in that** the presence or absence of at least three or at least four different mRNAs is recognized.

4. Procedure in accordance with one of the preceding claims, **characterized in that** the mRNA associated with the gene of CA12-5 is recognized.

5. Procedure in accordance with one of the preceding claims, **characterized in that** tumor cells from the blood sample are separated or concentrated, and the detection procedure is done using these tumor cells.

6. Procedure in accordance with the preceding claim, **characterized in that** the ovarian carcinoma cells are separated or concentrated by means of antibodies or antibody derivatives which are generally specific for tumor cells, and/or by means of antibodies or antibody derivatives which are specific for ovarian carcinoma cells, or by means of mixtures of such antibodies and/or antibody derivatives.

7. Procedure in accordance with the preceding claim, **characterized in that** the antibodies or antibody derivatives used for separating ovarian tumor cells have binding sites that bind to the epitopes of an epithelial antigen, of an epithelial membrane antigen and/or of the antigen MUC1.

8. Procedure in accordance with the preceding claim, **characterized in that** use is made of at least one antibody of the group comprising antibodies 2D3, MOC-31, 10E9 and HMPV, or of a mixture of two, three or four of these antibodies, as the antibody.

9. Procedure in accordance with one of claims 6 to 8, **characterized in that** the ovarian carcinoma cells are separated or concentrated by means of antibodies bound to magnetic particles.

10. Procedure in accordance with one of claims 6 to 9, **characterized in that** the ovarian carcinoma cells are separated or concentrated by means of fluorescence-activated continuous flow cytometry, density gradient centrifugation, and/or centrifugation following erythrocyte lysis.

11. Procedure in accordance with Claim 10, **characterized in that** leukocytes in the blood sample are pelletized by centrifugation.

12. Procedure in accordance with Claim 10, **characterized in that** the RNA-containing components of the sample are concentrated via lysis of the erythrocytes that are contained in it, together with subsequent pelletization of the nonlysed leukocytes.

13. Procedure in accordance with Claim 10, **characterized in that** the RNA-containing components are concentrated by at least one density gradient centrifugation of the blood sample in order to separate and harvest the mononuclear blood cells that are contained in it.

14. Procedure in accordance with one of Claims 10 through 13, **characterized in that** the harvested mononuclear blood cells are labeled with fluorescence-labeled antibodies or antibody derivatives, and are separated and harvested by means of fluorescence-activated cell sorting (FACTS) of the sample.

15. Procedure in accordance with one of Claims 10 through 14, **characterized in that** the mononuclear cells from the harvested fraction are lysed, and the mRNA is separated.

16. Procedure in accordance with one of claims 1 to 4, **characterized in that** the RNA (total RNA or mRNA) is isolated directly and in a conventional manner from the whole blood sample.

17. Procedure in accordance with the preceding claim, **characterized in that** DNA digestion is carried out subsequently to isolation of the RNA.

18. Procedure in accordance with one of the preceding claims, **characterized in that** the harvested mRNA is reverse transcribed into cDNA, and the presence or absence of the cDNA that is assigned to the tumor marker protein is recognized.

19. Procedure in accordance with the preceding claim, **characterized in that** at least one predetermined segment of the cDNA is replicated by means of polymerase chain reaction ("PCR").

20. Procedure in accordance with the preceding claim, **characterized in that** one or more oligonucleotide pairs from the group of pairs which comprise or consist of the sequences of the following group of sequences are used for replicating the cDNA, the group comprising the following pairs of sequences:
AATCGTCAATGCCAGTGTACTTCA and
TAACGCGTTGTGATCTCCTTCTGA,
AGTCGGGCTCTGGAGGAAAAGAAA and
GATCATAATTCCTCTGCACATAGG,
AGAAATGACGCAAGAGCCTATGTA and
AACTTGTGTGTGTTGCTGCGGTAT,
TCAGCTTCTACTCTGGTGCACAAC and
TGGTAGTAGTCGGTGCTGGGATCT,
CCCAGTGTGTCAACTGCAGCCAGT and
CAGATGGGCATGTAGGAGAGGTCA,
TCAACGCCTCGGTGGCATAC and
CTGTCACATCGCGCACGTCT,
ACACATTCCGCTTCTGCCTGGTCA and
CCCCGGTGTGGTGCCTGTT

21. Procedure in accordance with one of the preceding claims, **characterized in that** the mRNA of the protein β-actin is determined for internal control purposes.

22. Procedure in accordance with the preceding claim, **characterized in that** the mRNA for β-actin is reverse transcribed into cDNA, and a segment of the cDNA is replicated by means of a polymerase chain reaction.

23. Procedure in accordance with the preceding claim, **characterized in that** an oligonucleotide pair is used for replicating the cDNA of the β-actin, whereby the oligonucleotides of the pair comprise or consist of the following sequences:
CTG GAG AAG AGC TAC GAG CTG CCT and
ACA GGA CTC CAT GCC CAG GAA GGA.

24. Procedure in accordance with one of Claims 19 through 23, **characterized in that** the replicated cDNA segment is digested via suitable restriction enzymes, and the presence or absence of the mRNA coding for a tumor marker protein is determined by means of the cDNA fragments that are produced.

25. Procedure in accordance with one of Claims 19 through 24, **characterized in that** gel electrophoresis of the PCR products is carried out in order to detect the amplified cDNA segments.

26. Procedure in accordance with one of Claims 19 through 24, **characterized in that** fragment analysis is carried out in order to detect the amplified cDNA segments.

27. Procedure in accordance with one of Claims 19 through 24, **characterized in that,** during the course of the polymerase chain reaction, the fluorescence produced by the products is recognized, and the formation of products is recognized (fluorescence-based real time PCR).

28. Procedure in accordance with one of Claims 19 through 24, **characterized in that** use is made of a nucleotide microarray in accordance with one of Claims 48 to 50 in order to detect the mRNA or cDNA.

29. Procedure in accordance with the preceding claim, **characterized in that** the PCR product is applied to a nucleotide microarray in accordance with one of Claims 48 to 50 in order to detect the amplified cDNA.

30. Diagnosis kit for the diagnosis or monitoring of ovarian cancer via at least two different pairs of oligonucleotides (reverse primers, forward primers), whereby the two oligonucleotides of each pair are suitable as primers for amplification by means of a polymerase chain reaction of, in each case, one of the two complementary strands of for the two pairs different DNA segments that are being sought, and whereby the DNA segments that are being sought are selected from the group comprising as members cDNA associated with the genes EGFR, CEA, GA733-2, MUC1, HER2, CA12-5 and MUC4.

31. Diagnosis kit in accordance with Claim 30, **characterized in that** it contains at least three different pairs of oligonucleotides (reverse primers, forward primers), whereby the two oligonucleotides of each pair are suitable as primers for amplification by means of a polymerase chain reaction of, in each case, one of the two complementary strands of for the three pairs each different DNA segments that are being sought, and whereby the DNA segments that are being sought are selected from the group comprising as members cDNA associated with various tumor marker proteins EGFR, CEA, GA733-2, MUC1, HER2, CA12-5 and MUC4.

32. Diagnosis kit in accordance with Claim 30 or Claim 31, **characterized in that** the DNA segments that are being sought comprise members of at least one group of the following groups of marker combinations:
GA733-2 and MUC1;
GA733-2 and HER2;
GA733-2 and CEA;
GA733-2 and CA12-5;
GA733-2 and EGFR;
GA733-2 and MUC4;
or
GA733-2, MUC1 and HER2;
GA733-2, MUC1 and CA12-5;
GA733-2, MUC1 and EGFR;
GA733-2, MUC1 and CEA;
GA733-2, MUC1 and MUC4;
GA733-2, CA12-5 and EGFR;
GA733-2, CA12-5 and CEA;
GA733-2, CA12-5 and HER2;
GA733-2, CA12-5 and MUC4;
GA733-2, HER2 and EGFR;
GA733-2, HER2 and CEA;
GA733-2, HER2 and MUC4;
GA733-2, EGFR and CEA;
GA733-2, EGFR and MUC4;
GA733-2, CEA and MUC4;
or
GA733-2, HER2, CEA and EGFR;
GA733-2, HER2, MUC1 and CA12-5;
GA733-2, HER2, MUC1, CA12-5, EGFR and CEA;
GA733-2, MUC1, CA12-5 and EGFR;
GA733-2, HER2, MUC4 and EGFR;
GA733-2, HER2, MUC4 and CEA;
or further combinations of markers comprising at least marker GA733-2 as first marker, at least one marker of the group of markers EGRF, CEA, MUC1, HER2, CA12-5 and MUC4 as second marker, at least one marker of the group of markers EGRF, CEA, MUC1, HER2, CA12-5 and MUC4 as third marker different from the second marker and at least one marker of the group of markers EGRF, CEA, MUC1, HER2, CA12-5 and MUC4 as forth marker different from the second marker and different from the third marker.

33. Diagnosis kit in accordance with one of claims 30 to 32, **characterized in that** it contains at least four different pairs of oligonucleotides (reverse primers, forward primers), whereby the two oligonucleotides of each pair are suitable as primers for amplification by means of a polymerase chain reaction of, in each case, one of the two complementary strands of for the four pairs each different DNA segments that are being sought, and whereby the DNA segments that are being sought comprise the cDNA associated with tumor marker proteins selected from the group comprising tumor marker proteins EGFR, CEA, GA733-2, MUC1, HER2, CA12-5 and MUC4.

34. Diagnosis kit in accordance with one of Claims 30 to 33, **characterized in that** the two oligonucleotides of a pair from the group of pairs comprise or consist of one of the sequences of the following group of sequences in a pair-wise manner, the group of sequences comprising the following pairs of sequences:
AATCGTCAATGCCAGTGTACTTCA and
TAACGCGTTGTGATCTCCTTCTGA,
AGTCGGGCTCTGGAGGAAAAGAAA and
GATCATAATTCCTCTGCACATAGG,
AGAAATGACGCAAGAGCCTATGTA and
AACTTGTGTGTGTTGCTGCGGTAT,
TCAGCTTCTACTCTGGTGCACAAC and
TGGTAGTAGTCGGTGCTGGGATCT,
CCCAGTGTGTCAACTGCAGCCAGT and
CAGATGGGCATGTAGGAGAGGTCA,
TCAACGCCTCGGTGGCATAC and
CTGTCACATCGCGCACGTCT,
ACACATTCCGCTTCTGCCTGGTCA and
CCCCGGTGTGGTGCCTGTT.

35. Diagnosis kit in accordance with one of claims 30 to 34, **characterized in that** it contains an additional pair of oligonucleotides that are suitable as primers for the amplification of at least one segment of one of the two complementary strands of the cDNA associated with the protein β-actin for internal control purposes.

36. Diagnosis kit in accordance with the preceding claims, **characterized in that,** in order to amplify the cDNA associated with β-actin, it contains a pair of oligonucleotides comprising or consisting of the following sequences:
CTG GAG AAG AGC TAC GAG CTG CCT and
ACA GGA CTC CAT GCC CAG GAA GGA.

37. Diagnosis kit in accordance with one of claims 30 to 36, **characterized in that,** in each case, at least one of the two oligonucleotides of a pair of oligonucleotides is labeled with fluorophores.

38. Diagnosis kit in accordance with the preceding claim, **characterized in that** the oligonucleotides of different pairs are labeled with different fluorophores.

39. Diagnosis kit in accordance with one of Claims 30 to 38, **characterized in that** it contains the substances required for carrying out a polymerase chain reaction.

40. Diagnosis kit in accordance with the preceding claims, **characterized in that** it contains the following as the substances required for carrying out a polymerase chain reaction: a buffer solution, magnesium chloride, deoxynucleotide triphosphates as well as a heat-stable polymerase.

41. Diagnosis kit in accordance with the preceding claim, **characterized in that** it contains a polymerase from Thermus aquaticus (Taq polymerase) as the heat-stable polymerase.

42. Diagnosis kit in accordance with one of Claims 30 to 41, **characterized in that,** as a positive control, it contains a DNA sample with the DNA segment that is being sought in each case.

43. Diagnosis kit in accordance with one of Claims 30 to 42, **characterized in that** it contains:
instructions for carrying out the polymerase chain reaction and/or
instructions for carrying out a fragment analysis.

44. Diagnosis kit in accordance with one of Claims 30 to 43, **characterized in that** it contains a schematic arrangement for evaluating the measurement results.

45. Diagnosis kit in accordance with one of Claims 30 to 44, **characterized in that** it contains a microarray (DNA chip), whereby the array has a number of cells (fields) that are separated from one another, and an oligonucleotide, which hybridizes with the DNA segment that is being sought, is arranged in at least one cell of the microarray.

46. Diagnosis kit in accordance with the preceding claim, **characterized in that** an additional oligonucleotide arranged in at least one additional cell of the microarray, and the sequence of the nucleotide arranged in said cell differs from the sequence of the additional oligonucleotide.

47. Diagnosis kit in accordance with one of the two preceding claims, **characterized in that,** in each case, an oligonucleotide is arranged in at least two cells, whereby the oligonucleotides arranged in different cells hybridize in each case with the different DNA segments that are being sought.

48. Microarray for the diagnosis or monitoring of ovarian cancer, e.g. a DNA chip, with an arrangement of several cells that are separated from one another, **characterized in that,** in each case, different oligonucleotides are arranged in at least two cells, whereby these oligonucleotides hybridize with a DNA segment that is part of the cDNA associated with two different members of the group comprising tumor marker proteins EGFR, CEA, GA733-2, MUC1, HER2, CA12-5 and MUC4.

49. Microarray in accordance with Claim 48, **characterized in that** different oligonucleotides are arranged in two to four cells in each case, whereby these oligonucleotides hybridize in each case with two to four different DNA segments, which are part of the cDNA of, in each case, various tumor marker proteins that are selected from the group comprising tumor marker proteins EGFR, CEA, GA733-2, MUC1, HER2, CA12-5 and MUC4.

50. Microarray in accordance with Claim 48 or Claim 49, **characterized in that** different oligonucleotides are, in each case, arranged in at least two cells, whereby these oligonucleotides hybridize with DNA segments that are part of the cDNA associated with genes from the groups of genes coding for tumor markers comprising the following groups of tumor marker combinations
GA733-2 and MUC1;
GA733-2 and HER2;
GA733-2 and CEA;
GA733-2 and CA12-5;
GA733-2 and EGFR;
GA733-2 and MUC4;
or
GA733-2, MUC1 and HER2;
GA733-2, MUC1 and CA12-5;
GA733-2, MUC1 and EGFR;
GA733-2, MUC1 and CEA;
GA733-2, MUC1 and MUC4;
GA733-2, CA12-5 and EGFR;
GA733-2, CA12-5 and CEA;
GA733-2, CA12-5 and HER2;
GA733-2, CA12-5 and MUC4;
GA733-2, HER2 and EGFR;
GA733-2, HER2 and CEA;
GA733-2, HER2 and MUC4;
GA733-2, EGFR and CEA;
GA733-2, EGFR and MUC4;
GA733-2, CEA and MUC4;
or
GA733-2, HER2, CEA and EGFR;
GA733-2, HER2, MUC1 and CA12-5;
GA733-2, HER2, MUC1, CA12-5, EGFR and CEA;
GA733-2, MUC1, CA12-5 and EGFR;
GA733-2, HER2, MUC4 and EGFR;
GA733-2, HER2, MUC4 and CEA;
or further combinations of markers comprising at least marker GA733-2 as first marker, at least one marker of the group of markers EGRF, CEA, MUC1, HER2, CA12-5 and MUC4 as second marker, at least one marker of the group of markers EGRF, CEA, MUC1, HER2, CA12-5 and MUC4 as third marker different from the second marker and at least one marker of the group of markers EGRF, CEA, MUC1, HER2, CA12-5 and MUC4 as forth marker different from the second marker and different from the third marker.

51. Use of a diagnosis kit, a microarray, and/or a procedure in accordance with one of the preceding claims for the diagnosis of diseases or metastasis, or for monitoring, in cases of ovarian cancer.

## Amended claims

### Amended claims in accordance with Rule 137(2) EPC.

**1.** Procedure for the detection of circulating ovarian tumor cells or monitoring of ovarian cancer in humans, **characterized in that** the presence or absence of at least two different mRNAs is recognized in a human blood sample, whereby these mRNAs code for at least two members of the group comprising tumor marker proteins EGFR, CEA, GA733-2, MUC1, HER2, CA12-5 and MUC4 and conclusions are drawn from this in regard to the presence of ovarian carcinoma cells in the sample of blood, and hence in regard to possible metastasis, wherein the sample is considered to be positive for the presence of ovarian carcinoma cells even if only one of the tumor markers to be recognized turns out to be present.

**2.** Procedure in accordance with Claim 1, **characterized in that** the presence or absence is recognized of members of at least one group of the following groups of marker:
GA733-2 and MUC1;
GA733-2 and HER2;
GA733-2 and CEA;
GA733-2 and CA12-5;
GA733-2 and EGFR;
GA733-2 and MUC4;
or
GA733-2, MUC1 and HER2;
GA733-2, MUC1 and CA12-5;
GA733-2, MUC1 and EGFR;
GA733-2, MUC1 and CEA;
GA733-2, MUC1 and MUC4;
GA733-2, CA12-5 and EGFR;
GA733-2, CA12-5 and CEA;
GA733-2, CA12-5 and HER2;
GA733-2, CA12-5 and MUC4;
GA733-2, HER2 and EGFR;
GA733-2, HER2 and CEA;
GA733-2, HER2 and MUC4;
GA733-2, EGFR and CEA;
GA733-2, EGFR and MUC4;
GA733-2, CEA and MUC4;
or
GA733-2, HER2, CEA and EGFR;
GA733-2, HER2, MUC1 and CA12-5;
GA733-2, HER2, MUC1, CA12-5, EGFR and CEA;
GA733-2, MUC1, CA12-5 and EGFR;
GA733-2, HER2, MUC4 and EGFR;
GA733-2, HER2, MUC4 and CEA;
or further combinations of at least markers comprising at least marker GA733-2 as first marker, at least one marker of the group of markers EGRF, CEA, MUC1, HER2, CA12-5 and MUC4 as second marker, at least one marker of the group of markers EGRF, CEA, MUC1, HER2, CA12-5 and MUC4 as third marker different from the second marker and at least one marker of the group of markers EGRF, CEA, MUC1, HER2, CA12-5 and MUC4 as forth marker different from the second marker and different from the third marker.

**3.** Procedure in accordance with one of the preceding claims, **characterized in that** the presence or absence of at least three or at least four different mRNAs is recognized.

**4.** Procedure in accordance with one of the preceding claims, **characterized in that** the mRNA associated with the gene of CA12-5 is recognized.

**5.** Procedure in accordance with one of the preceding claims, **characterized in that** tumor cells from the blood sample are separated or concentrated, and the detection procedure is done using these tumor cells.

**6.** Procedure in accordance with the preceding claim, **characterized in that** the ovarian carcinoma cells are separated or concentrated by means of antibodies or antibody derivatives which are generally specific for tumor cells, and/or by means of antibodies or antibody derivatives which are specific for ovarian carcinoma cells, or by means of mixtures of such antibodies and/or antibody derivatives.

**7.** Procedure in accordance with the preceding claim, **characterized in that** the antibodies or antibody derivatives used for separating ovarian tumor cells have binding sites that bind to the epitopes of an epithelial antigen, of an epithelial membrane antigen and/or of the antigen MUC1.

**8.** Procedure in accordance with the preceding claim, **characterized in that** use is made of at least one antibody of the group comprising antibodies 2D3, MOC-31, 10E9 and HMPV, or of a mixture of two, three or four of these antibodies, as the antibody.

**9.** Procedure in accordance with one of claims 6 to 8, **characterized in that** the ovarian carcinoma cells are separated or concentrated by means of antibodies bound to magnetic particles.

**10.** Procedure in accordance with one of claims 6 to 9, **characterized in that** the ovarian carcinoma cells are separated or concentrated by means of fluorescence-activated continuous flow cytometry, density gradient centrifugation, and/or centrifugation following erythrocyte lysis.

**11.** Procedure in accordance with claim 10, **characterized in that** leukocytes in the blood sample are pelletized by centrifugation.

**12.** Procedure in accordance with claim 10, **characterized in that** the RNA-containing components of the sample are concentrated via lysis of the erythrocytes that are contained in it, together with subsequent pelletization of the nonlysed leukocytes.

**13.** Procedure in accordance with claim 10, **characterized in that** the RNA-containing components are concentrated by at least one density gradient centrifugation of the blood sample in order to separate and harvest the mononuclear blood cells that are contained in it.

**14.** Procedure in accordance with one of claims 10 through 13, **characterized in that** the harvested mononuclear blood cells are labeled with fluorescence-labeled antibodies or antibody derivatives, and are separated and harvested by means of fluorescence-activated cell sorting (FACS) of the sample.

**15.** Procedure in accordance with one of claims 10 through 14, **characterized in that** the mononuclear cells from the harvested fraction are lysed, and the mRNA is separated.

**16.** Procedure in accordance with one of claims 1 to 4, **characterized in that** the RNA (total RNA or mRNA) is isolated directly and in a conventional manner from the whole blood sample.

**17.** Procedure in accordance with the preceding claim, **characterized in that** DNA digestion is carried out subsequently to isolation of the RNA.

**18.** Procedure in accordance with one of the preceding claims, **characterized in that** the harvested mRNA is reverse transcribed into cDNA, and the presence or absence of the cDNA that is assigned to the tumor marker protein is recognized.

**19.** Procedure in accordance with the preceding claim, **characterized in that** at least one predetermined segment of the cDNA is replicated by means of polymerase chain reaction ("PCR").

**20.** Procedure in accordance with the preceding claim, **characterized in that** one or more oligonucleotide pairs from the group of pairs which comprise or consist of the sequences of the following group of sequences are used for replicating the cDNA, the group comprising the following pairs of sequences:
AATCGTCAATGCCAGTGTACTTCAand
TAACGCGTTGTGATCTCCTTCTGA,
AGTCGGGCTCTGGAGGAAAAGAAAand
GATCATAATTCCTCTGCACATAGG,
AGAAATGACGCAAGAGCCTATGTA and
AACTTGTGTGTGTTGCTGCGGTAT,
TCAGCTTCTACTCTGGTGCACAAC and
TGGTAGTAGTCGGTGCTGGGATCT,
CCCAGTGTGTCAACTGCAGCCAGT and
CAGATGGGCATGTAGGAGAGGTCA,
TCAACGCCTCGGTGGCATAC and
CTGTCACATCGCGCACGTCT,
ACACATTCCGCTTCTGCCTGGTCA and
CCCCGGTGTGGTGCCTGTT.

**21.** Procedure in accordance with one of the preceding claims, **characterized in that** the mRNA of the protein β-actin is determined for internal control purposes.

**22.** Procedure in accordance with the preceding claim, **characterized in that** the mRNA for β-actin is reverse transcribed into cDNA, and a segment of the cDNA is replicated by means of a polymerase chain reaction.

**23.** Procedure in accordance with the preceding claim, **characterized in that** an oligonucleotide pair is used for replicating the cDNA of the β-actin, whereby the oligonucleotides of the pair comprise or consist of the following sequences:
CTG GAG AAG AGC TAC GAG CTG CCT and
ACA GGA CTC CAT GCC CAG GAA GGA.

**24.** Procedure in accordance with one of claims 19 through 23, **characterized in that** the replicated cDNA segment is digested via suitable restriction enzymes, and the presence or absence of the mRNA coding for a tumor marker protein is determined by means of the cDNA fragments that are produced.

**25.** Procedure in accordance with one of claims 19 through 24, **characterized in that** gel electrophoresis of the PCR products is carried out in order to detect the amplified cDNA segments.

**26.** Procedure in accordance with one of claims 19 through 24, **characterized in that** fragment analysis is carried out in order to detect the amplified cDNA segments.

**27.** Procedure in accordance with one of claims 19 through 24, **characterized in that**, during the course of the polymerase chain reaction, the fluorescence produced by the products is recognized, and the formation of products is recognized (fluorescence-based real time PCR).

**28.** Procedure in accordance with one of claims 19 through 24, **characterized in that**, in order to detect the mRNA or cDNA, use is made of a nucleotide microarray, wherein said microarray, e.g. a DNA chip, comprises an arrangement of several cells that are separated from one another, wherein in each case, different oligonucleotides are arranged in at least two cells, whereby these oligonucleotides hybridize with a DNA segment that is part of the cDNA associated with two different members of the group comprising tumor marker proteins EGFR, CEA, GA733-2, MUC1, HER2, CA12-5 and MUC4.

**29.** Procedure in accordance with claim 28, **characterized in that** in the microarray different oligonucleotides are arranged in two to four cells in each case, whereby these oligonucleotides hybridize in each case with two to four different DNA segments, which are part of the cDNA of, in each case, various tumor marker proteins that are selected from the group comprising tumor marker proteins EGFR, CEA, GA733-2, MUC1, HER2, CA12-5 and MUC4.

**30.** Procedure in accordance with claim 28 or claim 29, **characterized in that** in the microarray different oligonucleotides are, in each case, arranged in at least two cells, whereby these oligonucleotides hybridize with DNA segments that are part of the cDNA associated with genes from the groups of genes coding for tumor markers comprising the following groups of tumor marker combinations
GA733-2 and MUC1;
GA733-2 and HER2;
GA733-2 and CEA;
GA733-2 and CA12-5;
GA733-2 and EGFR;
GA733-2 and MUC4;
or
GA733-2, MUC1 and HER2;
GA733-2, MUC1 and CA12-5;
GA733-2, MUC1 and EGFR;
GA733-2, MUC1 and CEA;
GA733-2, MUC1 and MUC4;
GA733-2, CA12-5 and EGFR;
GA733-2, CA12-5 and CEA;
GA733-2, CA12-5 and HER2;
GA733-2, CA12-5 and MUC4;
GA733-2, HER2 and EGFR;
GA733-2, HER2 and CEA;
GA733-2, HER2 and MUC4;
GA733-2, EGFR and CEA;
GA733-2, EGFR and MUC4;
GA733-2, CEA and MUC4;
or
GA733-2, HER2, CEA and EGFR;
GA733-2, HER2, MUC1 and CA12-5;
GA733-2, HER2, MUC1, CA12-5, EGFR and CEA;
GA733-2, MUC1, CA12-5 and EGFR;
GA733-2, HER2, MUC4 and EGFR;
GA733-2, HER2, MUC4 and CEA;
or further combinations of markers comprising at least marker GA733-2 as first marker, at least one marker of the group of markers EGRF, CEA, MUC1, HER2, CA12-5 and MUC4 as second marker, at least one marker of the group of markers EGRF, CEA, MUC1, HER2, CA12-5 and MUC4 as third marker different from the second marker and at least one marker of the group of markers EGRF, CEA, MUC1, HER2, CA12-5 and MUC4 as forth marker different from the second marker and different from the third marker.

**31.** Procedure in accordance with one of claims 28 to 30, **characterized in that** the PCR product is applied to the nucleotide microarray in order to detect the amplified cDNA.

**32.** Use of a procedure in accordance with one of the preceding claims for the diagnosis of diseases or metastasis, or for monitoring, in cases of ovarian cancer.
